# EUROPEAN PATENT APPLICATION

(11) **EP 4 670 654 A1**
(43) Date of publication of application: **31.12.2025**
(21) Application number: 24777681.8
(22) Date of filing: 08.03.2024
(51) Int. Cl.: A61B 17/221, A61B 17/22

(54) **EMBOLUS RETRIEVAL DEVICE AND EMBOLUS RETRIEVER**

(30) Priority: 28.03.2023 CN 202310316027
(71) Applicant: Shanghai MicroPort Medical (Group) Co., Ltd., Shanghai 201203 (CN)
(72) Inventor: GENG, Kangkang, Shanghai 201203 (CN); ZHOU, Qi, Shanghai 201203 (CN); WU, Jintian, Shanghai 201203 (CN); HUANG, Haiyong, Shanghai 201203 (CN)
(74) Representative: Patentship Patentanwaltsgesellschaft mbH
(86) International application number: PCT/CN2024/080682
(87) International publication number: WO 2024/198891

(57) **Abstract**

The present invention provides a thrombectomy apparatus and device. The thrombectomy device includes a thrombectomy component and a control component. The thrombectomy component includes a thrombectomy stent including an outer mesh stent and an inner mesh stent nested within the outer mesh stent. The control component includes a movement control member and a mounting member. The movement control member is passed through the mounting member and extends out of a distal end thereof and then is coupled to a distal end of the thrombectomy stent, and the outer mesh stent is proximally attached to the mounting member. A proximal end of the inner mesh stent is attached to the mounting member or to the movement control member. The movement control member is able to control and change a diameter of the thrombectomy stent during movement of the movement control member toward a proximal or distal end of the thrombectomy device, adapting the thrombectomy device to various lumens. This can reduce possible damage to a lumen while allowing for effective removal of occlusive material.

## Description

### TECHNICAL FIELD

The present invention relates to the field of medical devices, and more particularly to a thrombectomy apparatus and device.

### BACKGROUND

An embolism refers to the phenomenon where an insoluble abnormal material enters the circulating blood, flows with the blood, and subsequently blocks the lumen of a blood vessel. A pulmonary embolism (PE) is a blockage of a pulmonary artery or a branch thereof by a detached blood clot or other material, which impairs pulmonary circulation. PEs are characterized by high incidence, high mortality, high recurrence, frequent missed diagnosis, etc. After the pulmonary artery or branch thereof is blocked, the effects of mechanical blockage and neurohumoral factors would lead to increased pulmonary circulation resistance, higher pulmonary arterial pressure, and abnormal ventilation-perfusion ratio, which may in turn cause a series of changes including right heart failure, decreased systemic blood pressure, congestion and severe hypoxemia. In severe cases, pulmonary infarction, pulmonary collapse, impaired functions of the heart, brain, kidneys and other important organs, or even sudden death may occur.

Conventional treatment of thromboembolisms and/or PEs involves reducing and/or removing abnormal material mainly by, among others, pharmaceutical anticoagulation, surgery or minimally invasive intervention. Pharmaceutical anticoagulation is conservative and suitable for patients with mild symptoms. However, patients with severe symptoms, who have failed medication, have to undergo a necessary surgical procedure. Compared with traditional surgical treatment, minimally invasive intervention features minimal trauma and fast recovery and is therefore regarded as a very promising technique for the treatment of acute embolisms or PEs. Minimally invasive intervention often employs approaches such as thrombolysis, rotational atherectomy, aspiration, basket, stent, or balloon thrombectomy to remove thrombus and other abnormal materials, thereby restoring blood flow within the vessel lumen. Among these approaches, stent or basket-based thrombectomy devices have been recognized by patients and surgeons and become an intensively studied topic of research in recent years because of excellent clinical outcomes. By establishing an access sheath, a thrombectomy device or a basket may be advanced to a thrombus site inside a blood vessel. After that, it may be released so as to penetrate the blood clot and then withdrawn therewith out of the body through the access sheath.

Conventional components for treating clot include a plurality of capture elements in the form of self-expanding disc-shaped units, which are radially or transversely raised outwards. Such disc-shaped units are typically braided from shape memory metal wires and able to effectively remove a fresh thrombus from a blood vessel while effectively preventing escape of disrupted thrombus. However, they are less effective for long-standing chronic clots adhering firmly to blood vessel walls. Additionally, when retracted into a catheter for withdrawal, the disc-shaped capture units may get stuck and piled up due to a small lumen of the catheter, impeding smooth retraction of the entire component for treating clot into the catheter and possibly leading to dislodgement and distal escape of a captured thrombus. In clinical practice, some thrombi are in the vicinity of branches in the lungs, which have tortuous lumens, and the lumen diameter varies considerably. When distally advanced into such a branch for thrombectomy, the disc-shaped units will self-expand to an outer diameter much larger than a diameter of the branch, resulting in high force against the branch vessel, leading to higher likelihood of causing damage to the blood vessel. There are also some conventional systems for treating clots, which include disc-shaped capture elements with cutouts formed by laser cutting or otherwise. These systems can provide great radial support and remove old thrombi. However, despite some capabilities of thrombus trapping, there is still a chance for distal escape of thrombus fragments through the capture elements, especially in the case of removing a fresh and soft thrombus. Further, when used to remove a stone from a bile duct, ureter or the like, the conventional devices would present problems similar to those with thrombus removal.It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY OF THE INVENTION

In order to overcome at least one of the above described problems associated with the prior art, the present invention provides a thrombectomy apparatus and device, which can effectively remove occlusive material from a blood vessel while causing reduced damage thereto.

To the above end, the present invention provides a thrombectomy device comprising a thrombectomy component and a control component. The thrombectomy component comprises a thrombectomy stent comprising an outer mesh stent and an inner mesh stent nested within the outer mesh stent. The control component comprises a movement control member and a mounting member. The movement control member is passed through the mounting member and extends out of a distal end thereof and is then coupled to a distal end of the thrombectomy stent. A proximal end of the outer mesh stent is attached to the mounting member, and a proximal end of the inner mesh stent is attached to the mounting member or to the movement control member. The movement control member is able to control the thrombectomy stent to change a diameter thereof during movement of the movement control member toward a proximal or distal end of the thrombectomy device.

In one embodiment, the outer mesh stent is a cut stent and the inner mesh stent is a braided stent.

In one embodiment, the movement control member comprises a rod-like structure and an internal luminal channel axially extending therethrough.

In one embodiment, a proximal end of the mounting member is provided with a handle, and wherein a proximal end of the movement control member is slidably coupled to the handle.

In one embodiment, the handle is provided with a slidable control portion, wherein the proximal end of the movement control member is coupled to the slidable control portion, and wherein the slidable control portion is configured to drive the movement control member to move.

In one embodiment, the slidable control portion comprises a locking structure configured to restrict a movement of the movement control member, wherein the movement control member can be restricted at a plurality of positions by the locking structure in a direction of movement, and wherein the plurality of positions corresponds to different diameters of the thrombectomy stent.

In one embodiment, the slidable control portion further comprises a slider and a slide track, wherein the slider is confined on the slide track so as to move along the slide track, wherein the proximal end of the movement control member is coupled to the slider, wherein the locking structure comprises a first locking member arranged on the slider and a plurality of second locking members arranged on the slide track, wherein the plurality of second locking members are arranged along a movement direction of the slider, and wherein the first locking member is selectively engageable with at last one of the plurality of the second locking members to restrict a movement of the slider.

In one embodiment, the thrombectomy stent, when expanded, has a diameter increasing and then decreasing from a proximal end to a distal end and has a maximum diameter greater than or equal to a diameter of a target lumen.

In one embodiment, the outer mesh stent comprises a proximal connecting portion, a proximal tapered portion, an intermediate wall-contacting portion, a distal tapered portion and a distal connecting portion that are joined in sequence from a proximal end to a distal end along an axis of the outer mesh stent, wherein the proximal connecting portion is made up of a plurality of extension struts that are circumferentially arranged, wherein the plurality of extension struts are coupled to the mounting member, wherein the proximal tapered portion has a diameter gradually increasing from a proximal end to a distal end along the axis of the outer mesh stent, wherein the distal tapered portion has a diameter gradually decreasing from a proximal end to a distal end along the axis of the outer mesh stent, wherein the intermediate wall-contacting portion has a maximum diameter greater than or equal to the diameter of the target lumen, wherein a diameter of the distal connecting portion is smaller than the diameter of the distal tapered portion, and wherein the distal tapered portion is coupled to the movement control member.

In one embodiment, the inner mesh stent comprises a proximal extension part, a proximal mesh surface, a distal mesh surface and a distal extension part that are joined in sequence from a proximal end to a distal end along an axis of the inner mesh stent, wherein the proximal extension part is made up of a plurality of pulling struts that are circumferentially arranged, wherein the plurality of pulling struts are coupled to the mounting member or to the movement control member, wherein the proximal mesh surface has a diameter gradually increasing from a proximal end to a distal end along the axis of the inner mesh stent, wherein the distal mesh surface has a diameter gradually decreasing from a proximal end to a distal end along the axis of the inner mesh stent, wherein a diameter of the distal extension part is smaller than the diameter of the distal mesh surface, and wherein the distal extension part is coupled to the movement control member.

In one embodiment, a mesh opening density of the proximal mesh surface is lower than a mesh opening density of the distal mesh surface, wherein a proximal end of the proximal extension part is inwardly crimped onto the outer surface of the movement control member, and wherein the distal extension part is inwardly crimped onto the outer surface of the movement control member.

In one embodiment, radiopaque markers are provided at a maximum diameter of an expanded outer mesh stent, and wherein the plurality of radiopaque markers are provided on a single circumference of the outer mesh stent.

In one embodiment, the thrombectomy component comprises a single or a plurality of thrombectomy stents, wherein in case of a plurality of thrombectomy stents, the plurality of thrombectomy stents are arranged in sequence along an axis of the thrombectomy device and are connected as a single piece, wherein after being passed out of the distal end of the mounting member, the movement control member is successively through all the thrombectomy stents and is coupled to a distal end of a distal-most one of the thrombectomy stents, and wherein a proximal end of a proximal-most one of the thrombectomy stents is coupled to the control component..

In one embodiment, a mesh opening density of the plurality of thrombectomy stents gradually increases from a proximal end to a distal end.

In one embodiment, in case of a single thrombectomy stent, the thrombectomy stent is coupled at a proximal end and a distal end to the control component through radiopaque rings, and wherein in case of a plurality of thrombectomy stents, a proximal end of a proximal-most one of the thrombectomy stents is coupled to the control component through a radiopaque ring, and wherein a distal end of a distal-most one of the thrombectomy stents is coupled to the control component through a radiopaque ring.

In one implementation, the thrombectomy component further comprises a graft covering some mesh openings in the outer mesh stent.

To the above end, the present invention also provides a thrombectomy apparatus comprising a delivery device, an aspiration catheter and the thrombectomy device as defined above. The delivery device is configured to deliver the thrombectomy device to a target thrombectomy site, and the aspiration catheter is coupled to the delivery device and configured to apply a suction force via the delivery device for aspirating occlusive material in the target lumen.

The present invention has the following benefits:
It provides a thrombectomy device comprising a thrombectomy component and a control component. The thrombectomy component comprises a thrombectomy stent comprising an outer mesh stent and an inner mesh stent nested within the outer mesh stent. The control component comprises a movement control member and a mounting member. The movement control member is passed through the mounting member and extends out of a distal end thereof and is then coupled to a distal end of the thrombectomy stent. A proximal end of the outer mesh stent is attached to the mounting member, and A proximal end of the inner mesh stent is attached to the mounting member or to the movement control member. The movement control member is able to control the thrombectomy stent to change a diameter thereof during movement of the movement control member toward a proximal end or distal end of the thrombectomy device.

With this arrangement, the thrombectomy stent is of a double-layer design, which enables the thrombectomy device to effectively remove both fresh, soft and old, strongly adherent occlusive material with high thrombectomy efficiency and good thrombectomy performance. In particular, when used for thrombectomy in a pulmonary artery in the vicinity of a pulmonary branch, the free diameter adjustability of the thrombectomy stent in an expanded configuration can reduce possible damage to the blood vessel. Of course, in other applications, the diameter of the expanded thrombectomy stent can be adjusted in real time according to a varying diameter of a vascular lumen, enabling the thrombectomy stent to accommodate various conditions of the lumen. This can reduce possible damage to the blood vessel, while ensuring close contact of the outer mesh stent with a wall of the blood vessel, which is essential to effective capture and removal of occlusive material therefrom.

As the thrombectomy apparatus of the present invention are based on the same inventive concept as the thrombectomy device of the present invention and thus has all the advantages of the thrombectomy device, the advantages thereof are not repeated herein.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1a is a schematic illustration of a thrombectomy apparatus according to a first embodiment of the present invention;
Fig. 1b is a schematic illustration of a thrombectomy device according to the first embodiment of the present invention;
Fig. 2a shows a schematic internal structural view of a handle according to the first embodiment of the present invention;
Fig. 2b is a schematic illustration of a slider according to the first embodiment of the present invention;
Fig. 2c schematically illustrates a connection of a proximal end of a movement control member and the slider according to the first embodiment of the present invention;
Fig. 2d is a schematic structural view of the slider according to the first embodiment of the present invention, taken from another angle;
Fig. 2e is a schematic diagram showing engagement of engagement protrusions in engagement recesses in the slider according to the first embodiment of the present invention;
Fig. 3 shows a schematic elevation view of an outer mesh stent according to the first embodiment of the present invention;
Fig. 4 shows a schematic side view of the outer mesh stent according to the first embodiment of the present invention;
Fig. 5a schematic illustrates one exemplary radiopaque marker on the outer mesh stent according to the first embodiment of the present invention;
Fig. 5b schematic illustrates another exemplary radiopaque marker on the outer mesh stent according to the first embodiment of the present invention;
Fig. 6 shows a schematic elevation view of an inner mesh stent according to the first embodiment of the present invention;
Fig. 7a is a schematic illustration of the thrombectomy device according to the first embodiment of the present invention, which has been expanded to a relatively large diameter;
Fig. 7b is a schematic illustration of the thrombectomy device according to the first embodiment of the present invention, which has been expanded to a relatively small diameter;
Fig. 8a schematically illustrates a sheath and a delivery catheter according to the first embodiment of the present invention, which have been successively advanced over a guide wire to a target thrombus during a surgical procedure;
Fig. 8b schematically illustrates the thrombectomy device according to the first embodiment of the present invention, which has been completely released and undergone an expansion during the surgical procedure;
Fig. 8c schematically illustrates a thrombectomy stent according to the first embodiment of the present invention, which has been adjusted by manipulating the handle to such a diameter that it comes into contact with a wall during the surgical procedure;
Fig. 8d schematically illustrates the removal of thrombus during the surgical procedure by retracting a mounting member according to the first embodiment of the present invention;
Fig. 9 shows a schematic illustration of a thrombectomy stent with a graft according to a second embodiment of the present invention; and
Fig. 10 shows a schematic illustration of multiple thrombectomy stents according to a third embodiment of the present invention, which are axially joined into a single integral piece.

### List of Reference Numerals

100-delivery device; 110-sheath; 110a-distal opening of sheath; 120-delivery catheter; 130-coupling base; 140-sealing valve for preventing blood leakage; 150-guide wire; 200-thrombectomy device; 210-thrombectomy stent; 211-inner mesh stent; 2111-pulling strut; 2112-proximal convergence end; 2113-proximal mesh surface; 2114-distal mesh surface; 2115-distal extension part; 212-outer mesh stent; 2121-extension strut; 2122-proximal tapered portion; 2123-distal tapered portion; 2124-intermediate wall-contacting portion; 2125-distal connecting portion; 2126-mesh opening of outer mesh stent; 2127-strut; 2128-intersection; 2129-outer connecting portion; 213radiopaque dot; 214-lateral strut; 215-radiopaque wire; 220-control component; 221-movement control member; 222-mounting member; 223-guide head; 2231-step; 224-handle; 225-slidable control portion; 2251-slider; 2252-slide track; 2253-backing plate; 2254-wall surface of slider; 2255-engagement recess; 2256-engagement protrusion; 226-radiopaque ring; 230-graft; 300-aspiration catheter; 310-one-way valve; 10-blood vessel; 20-target thrombus.

### DETAILED DESCRIPTION

Objects, advantages and features of the present invention will become more apparent upon reading the following more detailed description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales.

In this specification, the terms "proximal end" and "distal end" may be used to describe relative orientations, positions and directions of components of a medical device or actions taken thereon, as viewed by a surgeon operating the device. Without wishing to be limiting, "distal end" usually refers to an end of the medical device that enters the body of a patient first, and "proximal end" to an end closer to the surgeon, during normal operation of the device. In the context herein, "distal end" and "proximal end" refer to relative locations, rather than exact end of the structure. For instance, a "distal end" of a thrombectomy stent refers to a location near the end thereof, rather than exactly the end. As used herein, the term "axial direction" usually refers to a direction along a central axis, e.g., of a thrombectomy stent. "Radial direction" usually refers to a direction perpendicular to an axial direction. "Circumferential direction" refers to a direction about a central axis. "Advancement" refers to distal movement, while "retraction" or "withdrawal" refers to proximal movement. "Leading" means more proximal, while trailing means more distal. As used herein, a "target lumen" may be a natural lumen of a human, such as the lumen of a blood vessel, bile duct or ureter.

It is a principal object of the present invention to provide a thrombectomy apparatus and device, which overcome the problems associated with conventional apparatuses for thrombectomy and stone removal, including low efficiency, low capture success and a lack of adaptiveness to various lumens.

The present invention will be described below with reference to the accompanying drawings. In the following, should there be no conflict, the embodiments described hereunder and features thereof can complement or be combined with each other or one another.

### <Embodiment 1>

Description is set forth below in the context of removing a thrombus from a blood vessel. However, those skilled in the art can adapt the following description to the removal of a stone through appropriately modifying the details given below.

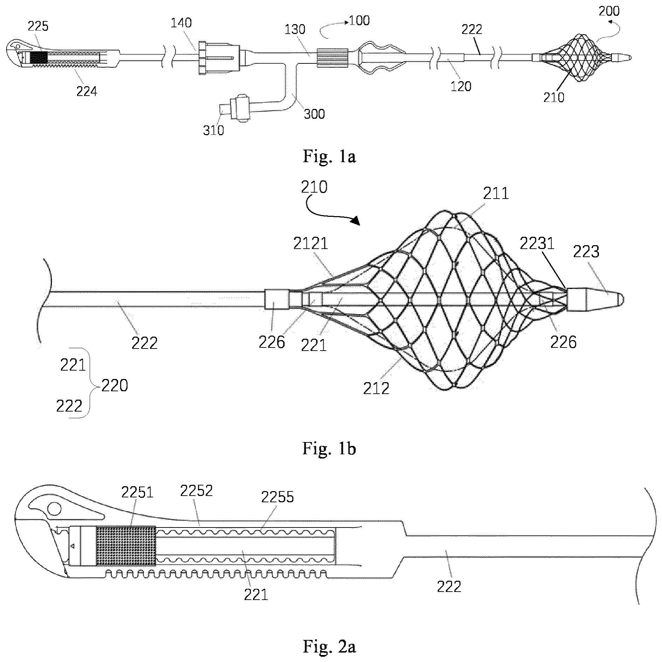

As shown in Figs. 1a and 1b, in a first embodiment of the present invention, there is provided a thrombectomy apparatus capable of interventional treatment of occlusive material. The thrombectomy apparatus includes a delivery device 100 and a thrombectomy device 200. The delivery device 100 is used to deliver the thrombectomy device 200 to a target site in need of thrombectomy. The thrombectomy device 200 includes a thrombectomy component and a control component 220. The thrombectomy component includes at last one thrombectomy stent 210. In one implementation, one thrombectomy stent 210 is included. The control component 220 is coupled to the thrombectomy stent 210, in order to be able to control release and retraction of the thrombectomy stent 210 and to vary a diameter of the thrombectomy stent 210.

The thrombectomy stent 210 includes an inner mesh stent 211 and an outer mesh stent 212. The inner mesh stent 211 is nested within the outer mesh stent 212. This double-layer design enables the thrombectomy device 200 not only to remove both old, strongly adherent and fresh, soft occlusive materials with high thrombectomy efficiency and good thrombectomy performance. Meanwhile, this double-layer stent can better capture a thrombus with a reduced risk of escape of captured thrombus and hence a reduced risk of occlusion caused by such escape of thrombus material.

The inner mesh stent 211 generally has a greater mesh opening density, i.e., denser meshing openings, than the outer mesh stent 212. The denser mesh openings enable to better block escape of thrombus. The outer mesh stent 212 is sparser compared to the inner mesh stent 211, and the sparser mesh openings allow to better cut and disrupt large thrombus.

The control component 220 includes a movement control member 221 and a mounting member 222. The movement control member 221 is passed through and extends out of the mounting member 222 from a distal end thereof and is then attached to a distal end of the thrombectomy stent 210. Specifically, distal ends of the outer mesh stent 212 and the inner mesh stent 211 are both fixedly connected to the movement control member 221, in order to enable the movement control member 221 to act on the distal end of the thrombectomy stent 210 to adjust diameter of the thrombectomy stent 210. Additionally, the outer mesh stent 212 is proximally and fixedly connected to the mounting member 222, and the proximal end of the inner mesh stent 211 is fixedly connected to the mounting member 222, or to the movement control member 221. In order to adjust the diameter of the thrombectomy stent 210 when the latter is in an expanded configuration, the mounting member 222 may be held stationary, while the movement control member 221 may be advanced or retracted relative to the mounting member 222, causing a change in the diameter of the thrombectomy stent 210. It will be understood that in the case of the inner mesh stent 211 being coupled to the movement control member 221 both proximally and distally, the outer mesh stent 212 will also act on the inner mesh stent 211, indirectly causing the inner mesh stent 211 to change its diameter along with the outer mesh stent 212.

With this arrangement, the diameter of the thrombectomy stent 210 in an expanded configuration can be easily adjusted in real time in response to lumen diameter changes of a blood vessel, making the thrombectomy stent 210 able to better accommodate various conditions of the lumen, with less damage to the blood vessel. Meanwhile, the outer mesh stent 212 can be brought into close contact with a wall of the blood vessel, which allows effective removal of occlusive material that adheres to the wall with high efficiency and good performance. In particular, when used for thrombectomy in a pulmonary artery in the vicinity of a pulmonary branch, the free diameter adjustability of the thrombectomy stent 210 in an expanded configuration can reduce possible damage to the blood vessel.

The outer mesh stent 212 is desired to have good radial support, which allows the thrombectomy stent 210 to, when in an expanded configuration, come into tighter contact with a luminal wall and thus effectively remove long-standing occlusive material that is hard and adheres to the luminal wall. In addition to sufficient radial support, the outer mesh stent 212 is also desired to have a large internal space capable of capturing a great amount of thrombus.

The inner mesh stent 211 is desired to have good compliance and adaptation, which allow it to easily change its diameter along with a diameter change of the outer mesh stent 212. The inner mesh stent 211 is able to effectively remove a fresh, soft thrombus from a lumen while effectively preventing escape of disrupted thrombus. Thus, once captured by the outer mesh stent 212, a thrombus can be additionally captured by the inner mesh stent 211 and prevented from escape, resulting in higher capture success.

In an optional implementation, the outer mesh stent 212 is a cut stent, and the inner mesh stent 211 is a braided stent. The braided stent provides better compliance and adaptation, and the cut stent provides better radial support.

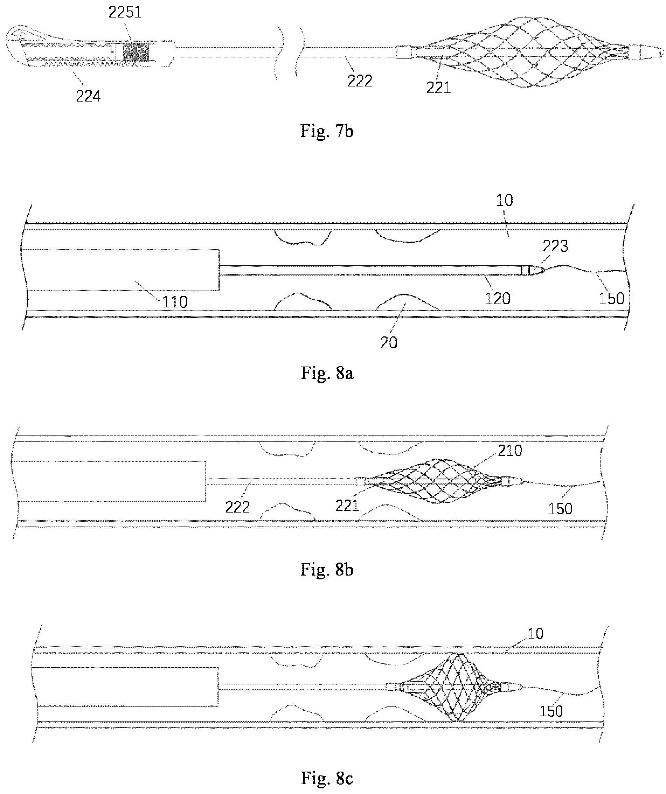

In one implementation, the movement control member 221 comprises a rod-like structure, and after it extends beyond the distal end of the mounting component 222, it can further support the thrombectomy stent 210, not only increasing its support strength but also restricting the movement direction of the thrombectomy stent 210 during diameter changes. The distal ends of the outer and inner mesh stent 212, 211 are both secured to the movement control member 221. Additionally, the movement control member 221 comprises an internal luminal channel axially extending therethrough, and a guide wire 150 (see Figs. 8a to 8d) or another auxiliary instrument can be passed through the channel. However, in other implementations, the rod-like movement control member 221 may be replaced with a pull string. In this case, the pull string can be loosened and tightened to change the diameter of the thrombectomy stent 210.

In the case of the movement control member 221 being implemented as a rod-like structure, a guide head 223 may be provided at its distal end to facilitate advancement of the thrombectomy device 200 within a blood vessel, with less damage to the blood vessel. The guide head 223 may be designed as a hollow cone with a spherical distal end face, which can not only provide guidance but can also reduce possible damage to a blood vessel. In addition, proximal end of the guide head 223 may comprise a step 2231, which can be used to limit a relative position between the thrombectomy device 200 and the delivery device 100 along the axial direction.

A proximal end of the movement control member 221 may be coupled to a proximal end of the mounting member 222, or not. In the former case, as shown in Figs. 1a and 2a, a handle 224 may be provided at the proximal end of the mounting member 222, and the proximal end of the movement control member 221 may be slidably coupled to the handle 224. In this case, an operator may actuate the movement control member 221 simply by manipulating the handle 224. In the case of the proximal end of the movement control member 221 not being coupled to the proximal end of the mounting member 222, an operator may activate the movement control member 221 by directly manipulating its proximal end. Regardless of the method, it is only necessary that the movement control member 221 can slide axially relative to the mounting member 222.

As shown in Figs. 1a and 2a, in one implementation, the handle 224 includes a slidable control portion 225, which is coupled to the proximal end of the movement control member 221 in order to be able to drive the movement control member 221. In addition, the slidable control portion 225 can be manually manipulated.

As shown in Figs. 2a to 2e, in one implementation, the slidable control portion 225 includes a slider 2251 and a slide track 2252. The internal structure of the handle 224 can be directly constructed to form a slide track 2252, or the slide track 2252 can be separately assembled inside the handle 224. Movement of the slider 2251 is limited to only sliding along the slide track 2252, and the movement direction of the slider 2251 is the movement direction of the movement control member 221. The proximal end of the movement control member 221 is received in the handle 224 and attached to the slider 2251. With this arrangement, an operator can cause the movement control member 221 to move forward or backward simply by manually moving the slider 2251. This design is simple in structure and easy to operate. However, those skilled in the art will recognize that, apart from the slider and the slide track, there are also many mechanisms capable of causing translation of the movement control member 221. In practice, at last one of such mechanisms may be selected, such as a rack-and-pinion drive, a threaded screw drive, and a worm and worm wheel drive.

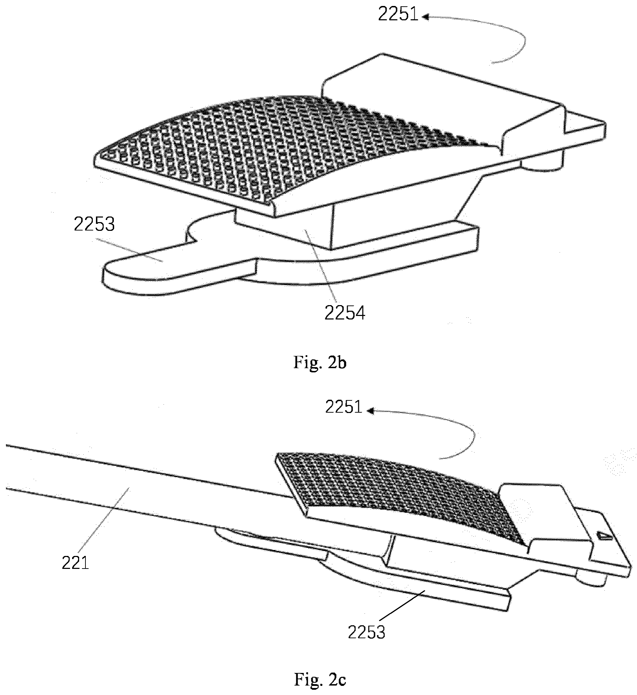

As shown in Figs. 2b and 2c, the slider 2251 may be provided with a backing plate 2253 configured to support part of the movement control member 221 to strengthen the coupling. The proximal end of the movement control member 221 is fixedly connected to a wall surface 2254 of the slider 2251. As a non-limiting example, the "fixedly connected" may be accomplished by welding, gluing or snap engagement. The slider 2251 may be provided with an anti-slip structure for increasing friction at the surface of the slider 2251 touched by hand.

Preferably, the slidable control portion 225 further includes a locking structure for restricting the movement of the slider 2251 and hence the movement control member 221 at a certain location from further movement. This enables more reliable and safer operations during surgery, reducing an operator's burden and increasing his/her convenience and comfort. The locking structure may stop the slider 2251 by force- or form-fitted locking at a plurality of positions in the direction of movement, the plurality of positions correspond to different diameters of the expanded thrombectomy stent 210. In one implementation, the locking structure includes a first locking member provided on the slider 2251 and a plurality of second locking members provided on the slide track 2252. The second locking members are arranged in the direction of movement of the slider 2251, and the first locking member can selectively engage one of the second locking members to stop the slider 2251 at a corresponding position.

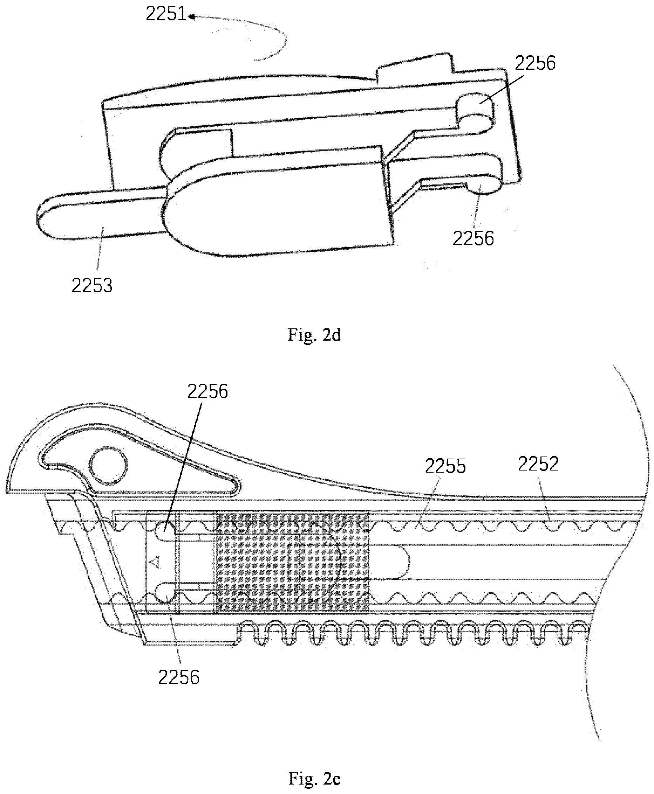

As shown in Figs. 2a, 2d and 2e, in one implementation, one of the first locking member and the second locking member is an engagement recess 2255, the other one is an engagement protrusion 2256. The engagement protrusion 2256 can engage the engagement recess 2255 to restrict the movement of the slider 2251. In this embodiment, the first locking member is an engagement protrusion 2256, and the second locking member is engagement recess 2255, the plurality of engagement recesses 2255 are arranged in the direction of movement of the slider 2251, the slider 2251 may stop at a position defined by the engagement recess 2255.

As shown in Figs. 2d and 2e, in one implementation, the slider 2251 are provide with two engagement protrusions 2256 distributed on two opposite sides (vertical or horizontal) of the slider 2251 perpendicular to the direction of movement. The two engagement protrusions 2256 can simultaneously engage in two symmetrical engagement recesses 2255. While the slider 2251 is being proximally or distally moved along the slide track 2252, the engagement protrusions 2256 will come into engagement with engagement recesses 2255 located at different positions. Such engagement can stop the slider 2251. However, the locking structure is not limited to being implemented by any of the above approaches. Indeed, those skilled in the art may select at least one of appropriate approaches known in the art.

As discussed above, the diameter of the expanded thrombectomy stent 210 can be controlled by adjusting the position of the movement control member 221 relative to the mounting member 222 through manipulating the slider 2251 on the handle 224, allowing the thrombectomy stent 210 to accommodate various conditions of a vascular lumen, thereby reducing possible damage to the blood vessel. Typically, a blood vessel has a smaller diameter at its distal end than at its proximal end. Accordingly, the diameter of the thrombectomy stent 210 may be reduced when it is advanced in a distal end of blood vessel. On the contrary, the diameter of the thrombectomy stent 210 may be increased when it is retracted in a proximal end of blood vessel.

The thrombectomy stent 210 may be self-expandable, or may be expanded under the action of external forces. In the former case, when leaving the delivery device 100, the thrombectomy stent 210 will expand itself, without aid of external force. After that, the control component 220 may be manipulated to additionally adjust the (outer) diameter of the thrombectomy stent 210. If the thrombectomy stent 210 is not of the self-expanding type, after pushed out of the delivery device 100, it further needs to be expanded with the aid of the control component 220. In this case, the control component 220 is used not only to expand the thrombectomy stent 210, but also to adjust the diameter of the expanded thrombectomy stent 210.

In one implementation, the thrombectomy stent 210 is a self-expanding stent optionally made of a metallic material, such as a metal or alloy, in particular with shape memory capabilities, examples of which may include, but are not limited to, nickel-titanium alloys. The inner mesh stent 211 and the outer mesh stent 212 may be made of the same or different materials.

Optionally, when in an expanded configuration, the diameter of the thrombectomy stent 210 may increase and then decrease from proximal end to distal enmd along axial direction thereof. This allows the thrombectomy stent 210 to be more stably guided back into the delivery device 100 with less effort while causing less damage to a blood vessel. In this case, the expanded thrombectomy stent 210 comprises a maximum diameter, which is greater than or equal to a diameter of a target vascular lumen. This ensures that the mesh surface at the expanded stent's maximum diameter can come into close contact with the vessel wall.

The distal end of the outer mesh stent 212 may be directly or indirectly attached to the movement control member 221. In one implementation, the distal end of the outer mesh stent 212 is directly attached to the guide head 223. It should be noted that the proximal and distal attachment of the outer mesh stent 212 may be accomplished by at least one of various approaches such as thermal fusion, adhesive bonding and mechanical coupling (e.g., welding, snap engagement, threaded coupling, etc.) In one implementation, the proximal end of the outer mesh stent 212 is attached to the mounting member 222 using a radiopaque ring 226. This radiopaque ring 226 at the proximal end of the outer mesh stent 212 helps determine the location of the proximal end and hence accurately locate the thrombectomy stent 210 within a blood vessel.

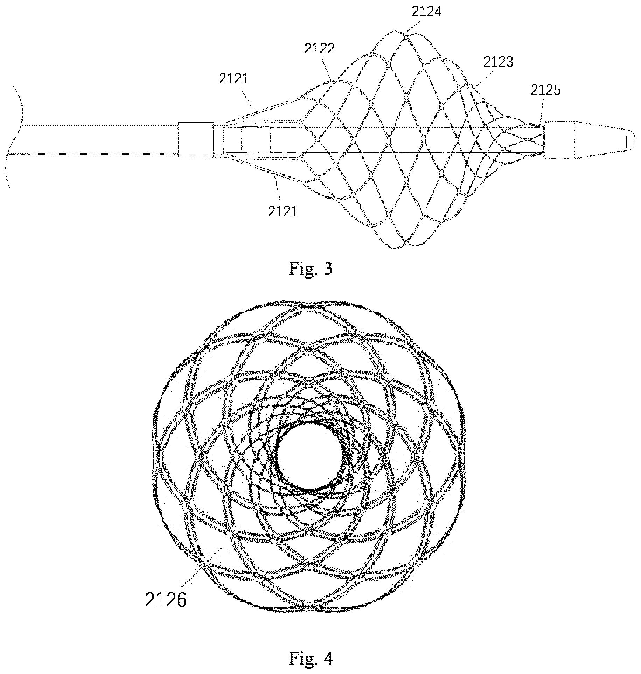

As shown in Figs. 3 and 4, the outer mesh stent 212 is in the shape of a mesh basket, which provides strong radial support while allows the outer mesh stent 212 to have a large accommodation space and provide better thrombus collection performance. Optionally, the outer mesh stent 212 is formed by cutting a shape memory metal. In this way, the resulting outer mesh stent 212 exhibits good radial support properties and can have large mesh openings to cut and disrupt a thrombus. In one implementation, the outer mesh stent 212 includes a proximal connecting portion and an outer mesh body. The proximal connecting portion located at a proximal end of the outer mesh body and serves to connect the mounting member 222 and to cut and disrupt a large thrombus. Shaped like a mesh basket, the outer mesh body serves to capture thrombus. The proximal connecting portion is made up of multiple circumferentially arranged extension struts 2121 fixedly connected to the mounting member 222. The number of extension struts 2121 may range from 2 to 4.

Optionally, the outer mesh body is configured to be, when expanded, larger in the middle and smaller at both ends. More specifically, the outer mesh body is distally closed and includes a proximal tapered portion 2122, an intermediate wall-contacting portion 2124 and a distal tapered portion 2123, which are joined in sequence from proximal to distal. The proximal connecting portion is joined to a proximal end of the proximal tapered portion 2122. The proximal tapered portion 2122 has a diameter gradually increasing from proximal to distal along an axis of the outer mesh stent 212. The distal tapered portion 2123 has a diameter gradually decreasing from proximal to distal along the axis of the outer mesh stent 212. Therefore, diameters of each of the proximal and distal tapered portions 2122, 2123 is smaller than the diameter of the intermediate wall-contacting portion 2124. A maximum diameter of the intermediate wall-contacting portion 2124 is greater than or equal to a diameter of a target vascular lumen, allowing the intermediate wall-contacting portion 2124 to come into contact with a wall of the blood vessel after expansion. Optionally, the proximal tapered portion 2122 may have a gentler taper than the distal tapered portion 2123. The outer mesh body further includes a distal connecting portion 2125 joined to a distal end of the distal tapered portion 2123, and a diameter of the distal connecting portion 2125 is smaller than a diameter of the distal tapered portion 2123, the distal tapered portion 2123. The distal connecting portion 2125 may be crimped tightly onto the outer surface of the movement control member 221 and then attached to the movement control member 221 directly or indirectly.

It will be understood that even when holding a captured thrombus, the outer mesh stent 212 still allows passage of blood therethrough. The multiple extension struts 2121 can comprise large mesh openings, which enable effective cutting and disruption of a large thrombus and direct the thrombus fragment into a space of the outer mesh body. Additionally, during retraction, the strong radial support of the outer mesh stent 212 allows it to effectively "scrape" and "peel" off adherent occlusive material, such as a thrombus, which is hard and adheres to a blood vessel wall. Further, the distal and proximal tapered portions of the outer mesh stent 212 can facilitate guiding of the thrombectomy stent 210 back into the delivery device 100, reducing effort required during withdrawal of the thrombectomy stent 210.

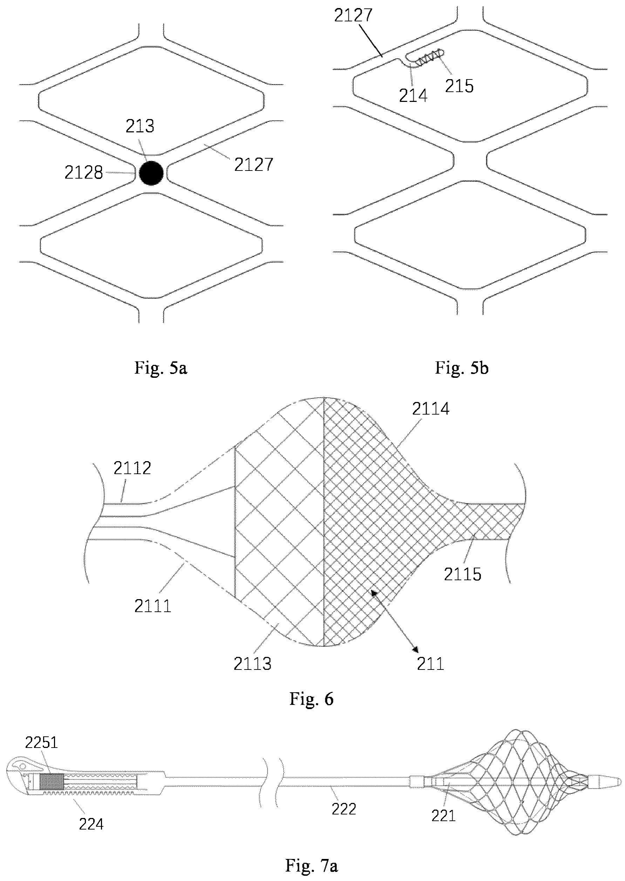

Optionally, the proximal tapered portion 2122 has a lower mesh opening density than the distal tapered portion 2123, enabling even better capture and trapping of thrombus. The present invention is not limited to any particular shape of mesh openings 2126 in the outer mesh body. In addition to rhombic openings, rectangular, circular, elliptic and other suitably shaped openings are also possible. As shown in Figs. 5a and 5b, the mesh openings 2126 in the outer mesh body are formed by connected struts 2127, and adjacent mesh openings 2126 are connected through intersections 2128. Joints of the struts 2127 and the intersections 2128 are rounded and smoothed to reduce stress concentration during expansion. The present invention is not limited to any particular shape of the intersections 2128, and one or a combination of rhombic, rectangular, circular and polygonal shapes is possible.

In the illustrated implementation, radiopaque markers are optionally provided on a location of the outer mesh body with a maximum diameter in an expanded configuration. The radiopaque markers may be fixedly attached to the outer mesh body in any suitable manner. In a fully expanded configuration of the outer mesh stent 212, the radiopaque markers may be evenly spaced on a single circumference of the outer mesh body. With these radiopaque markers, it is possible to monitor where the thrombectomy stent 210 is placed and how it contacts with a wall of a blood vessel in real time during a surgical procedure, thereby allowing adjusting in real time the location and diameter of the expanded thrombectomy stent 210 according to a lumen diameter of the blood vessel. In this way, close contact of the thrombectomy stent 210 with the blood vessel wall can be ensured throughout the surgical procedure. The radiopaque markers may be attached to the outer mesh stent 212, for example, by adhesive bonding or laser welding.

As shown in Fig. 5a, in one implementation, the radiopaque markers are radiopaque dots 213 disposed on intersections 2128. The radiopaque dots 213 may be, but is not limited to being, circular in shape. Of course, the radiopaque dots 213 may also be disposed at other locations than on the intersections 2128, as long as they are distributed on a single circumference to enable ensuring how the outer mesh stent 212 contacts a luminal wall. Alternatively, as shown in Fig. 5b, the radiopaque markers may be radiopaque wires 215 disposed on struts 2127. They may be directly wound on the struts 2127, or on lateral struts 214 projecting from one side of the struts 2127.Here, the radiopaque dots 213 and the radiopaque wires 215 are described merely as non-limiting examples.

As noted above, proximal end of the inner mesh stent 211 is attached to the mounting member 222, or to the movement control member 221. In one implementation, the proximal end of the inner mesh stent 211 is attached to the movement control member 221 by using a radiopaque ring 226. It should be noted that in the case of the proximal end of the inner mesh stent 211 being attached to the mounting member 222, the radiopaque ring 226 at the proximal end of the inner mesh stent 211 may be the same as the radiopaque ring 226 at the proximal end of the outer mesh stent 212, or may be a separate radiopaque ring. Additionally, the distal end of the inner mesh stent 211 is attached to the movement control member 221 with another radiopaque ring 226. The radiopaque ring 226 at the distal end of the inner mesh stent 211 can be used to determine the location of the distal end of the thrombectomy stent 210, facilitating accurate locating of the thrombectomy stent 210 within a blood vessel. However, in other implementations, the distal end of the inner mesh stent 211 may be fixedly connected to the guide head 223.

The proximal and distal attachment of the inner mesh stent 211 may be accomplished by at least one of various approaches such as thermal fusion, adhesive bonding and mechanical coupling (e.g., welding, snap engagement, threaded coupling, etc.) The proximal and distal attachment of the inner mesh stent 211 may be accomplished in the same manner as that of the outer mesh stent 212, or not.

Referring to Fig. 6, the inner mesh stent 211 is in the shape of a distally closed mesh cage, and when in an expanded configuration, its diameter is slightly smaller than that of the outer mesh stent 212, or the inner mesh stent contacts an inner wall of the outer mesh stent 212. In the illustrated implementation, the inner mesh stent 211 is braided from shape memory alloy wires. This imparts good compliance and adaptation and allows the formation of dense mesh openings. Structurally, the inner mesh stent 211 is similar to the outer mesh stent 212 and includes a proximal extension part and an inner mesh body. The proximal extension part is located at a proximal end of the inner mesh body and is configured for attachment to the mounting member 222 or the movement control member 221. Serving as a filter mesh, the inner mesh body serves to capture thrombus. The proximal extension part is made up of multiple circumferentially arranged pulling struts 2111, which are proximally attached to the mounting member 222 or to the movement control member 221. The proximal end of the multiple pulling struts 2111 forms a proximal convergence ends 2112 attached to the movement control member 221. The proximal convergence end 2112 can be inwardly gathered and tightly crimped onto the outer surface of the movement control member 221, imparting increased radial support.

In addition, the inner mesh body includes a proximal mesh surface 2113 and a distal mesh surface 2114 arranged along a proximal-to-distal direction. The proximal mesh surface 2113 and the distal mesh surface 2114 are joined to from a structure larger in the middle and smaller at both ends when in an expanded configuration. Thus, the proximal extension part, the proximal mesh surface 2113 and the distal mesh surface 2114 are connected in sequence along an axis of the inner mesh stent 211 from the proximal to distal end thereof. The proximal mesh surface 2113 has a diameter gradually increasing, and the distal mesh surface 2114 has a diameter gradually decreasing, along the axis of the inner mesh stent 211 from the proximal to distal end thereof. With this arrangement, the inner mesh stent 211 also comprises a shape facilitating retraction into the delivery device 100. Further, the proximal mesh surface 2113 has a lower mesh opening density than the distal mesh surface 2114. That is, the proximal mesh surface 2113 comprises sparser mesh opening and the distal mesh surface 2114 comprises denser mesh opening. The inner mesh body further includes a distal extension part 2115 having a smaller diameter than the distal mesh surface 2114 and attached to the movement control member 211. The distal extension part 2115 can be inwardly gathered to form a distal convergence end that is tightly crimped onto the outer surface of the movement control member 221, imparting improved radial support. With this arrangement, during retraction of the thrombectomy device 200, the outer mesh stent 212 can cut and disrupt a large thrombus, which are then received within a space of the inner mesh stent 211. Moreover, the denser mesh openings in the distal mesh surface 2114 of the inner mesh stent 211 can block a thrombus captured by the thrombectomy stent 210 from escape, avoiding otherwise possible occlusion.

Referring to Figs. 1a and 1b and 8a to 8d, in one implementation, the delivery device 100 includes a sheath 110 and a delivery catheter 120 in coaxial alignment with the sheath 110. The delivery catheter 120 is inserted through the sheath 110 and can be advanced therein. The thrombectomy device 200 can be crimped within the delivery catheter 120 and delivered to a target site in need of thrombectomy. The delivery catheter 120 may be provided at its proximal end with a coupling base 130 in the form of a Y-shaped connector. The Y-shaped connector has a proximal opening, where a flexible sealing valve 140 for preventing blood leakage is provided to seal off a proximal end of the coupling base 130. Alternatively, proximal ends of the delivery catheter 120 and the sheath 110 may also be sealed with such sealing valves.

In one implementation, the thrombectomy apparatus may further include an aspiration catheter 300 capable of facilitating removal of occlusive material. During thrombectomy, the aspiration catheter 300 may cooperate to suck thrombus out of the body, ensuring good thrombectomy efficiency and performance. To this end, the aspiration catheter 300 is configured to be able to aspirate a thrombus captured by the thrombectomy device 200. The aspiration catheter 30 may be detachably or non-detachably coupled to the coupling base 130 and is configured to apply a suction force via the delivery catheter 120 to aspirate a thrombus at a target lesion site. The aspiration catheter 300 is disposed on one side of the coupling base 130 and distally connected to a lateral hole in the coupling base 130. The proximal end of aspiration catheter 300 is often provided with a one-way valve 310 configured for connection with an external aspiration device. The aspiration device is able to apply a suction force to the delivery catheter 120 through the aspiration catheter 300. The aspiration device may be selected as any of possible fluid supply devices, for example, as a vacuum pump or the like.

Referring to Figs. 8a to 8d, a surgical access path may be first established with a sheath 110 along a guide wire 150, and after the thrombectomy device 200 is loaded in a crimped configuration within the delivery catheter 120, the delivery catheter 120 may then advance within the sheath 110. In this process, the step 2231 of the guide head 223 is provided outside the distal end of the delivery catheter 120 and is blocked by a distal end face of the delivery catheter 120, preventing the guide head 223 from entering the interior of the delivery catheter 120 and thereby restricting a relative position between the thrombectomy device 200 and the delivery catheter 120. The thrombectomy stent 210 can be then released simply by retracting the delivery catheter 120 relative to the thrombectomy device 200 or pushing the thrombectomy device 200 forward relative to the delivery catheter 120. Once the thrombectomy stent 210 is no longer confined by the delivery catheter 120 anymore, it transitions from the crimped configuration to an expanded configuration. In general cases, in order to release the thrombectomy stent 210, the delivery catheter 120 may be gradually retracted, concurrently with the thrombectomy device 200 being held stationary. On the contrary, when retracted, the thrombectomy device 200 can be pulled back, for example, generally into the sheath 110 during withdrawal.

Figs. 7a and 7b show adjustment of the diameter of the thrombectomy stent 210, for example, by manipulating the handle 224. As shown Fig. 7a, when the slider 2251 is located at a proximal-most position on the handle 224, the thrombectomy stent 210 is fully expanded to a maximum diameter. At this point, if the slider 2251 is slid distally, concurrently with the mounting member 222 being held stationary, the movement control member 221 will slide distally relative to the mounting member 222. As the outer mesh stent 212 is proximally attached to the mounting member 222 and distally to the movement control member 221, the distal end of the outer mesh stent 212 will distally move with the movement control member 221, thereby stretching and deforming the outer mesh stent 212, and a diameter of the softer outer mesh stent 212 is correspondingly reduced. At this time, the softer inner mesh stent 211 will be compressed by the outer mesh stent 212 and a diameter of the softer inner mesh stent 211 is also correspondingly reduced. This can adapt the stent to a blood vessel with a smaller diameter, in particular as shown in Fig. 7b. Likewise, the diameter of the thrombectomy stent 210 may be increased by sliding the slider 2251 to the proximal end, adapting it to a blood vessel with a larger diameter.

Figs. 8a to 8d show use of the thrombectomy apparatus of the present invention. As shown in Fig. 8a, first of all, a guide wire 150 is advanced up to a target thrombus 20 along a blood vessel 10. The guide wire 150 is then further advanced until its complete penetration through the target thrombus 20. After that, the sheath 100 is advanced over the guide wire 150 to the vicinity of the target thrombus 20 and then held stationary there. The delivery catheter 120, with the thrombectomy stent 210 having been crimped therein, is advanced over the guide wire 150 within the sheath 110 until the guide head 223 reaches a location more distal than the target thrombus 20. Subsequently, as shown in Fig. 8b, with the control component 220 being held stationary, the delivery catheter 120 is gradually retracted until the thrombectomy stent 210 is completely exposed. At this point, the thrombectomy stent 210 is in a configuration with a small diameter (and the slider 2251 is located at an initial position that is a distal-most possible position on the handle 224). As shown in Fig. 8c, by progressively adjusting the position of the slider 2251 toward the proximal end, the movement control member 221 retracts toward the proximal end of the mounting member 222, thereby gradually expanding the thrombectomy stent 210. In this process, an outer contour of the thrombectomy stent 210 within a lumen can be monitored in real time by observing the radiopaque markers, until its maximum diameter comes into close contact with a wall of the blood vessel.

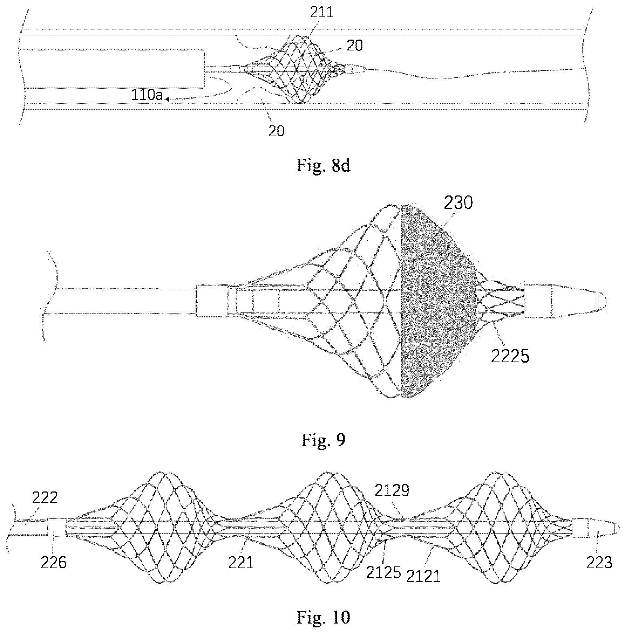

As shown in Fig. 8d, in order to remove the thrombus by the expanded thrombectomy stent 210, the mounting member 222 is gradually retracted proximally, causing the expanded thrombectomy stent 210 to slowly move toward the proximal end. In this process, the target thrombus 20 is gradually scraped, squeezed, cut and disrupted by the extension struts 2121, proximal tapered portion 2122 and the intermediate wall-contacting portion 2124 of the outer mesh stent 212 and the pulling struts 2111 and proximal mesh surface 2113 of the inner mesh stent 211 and is eventually received into the space of the inner mesh stent 211. Moreover, the distal mesh surface 2114 of the inner mesh stent 211 provides a space block structure for the thrombus, which enables the thrombectomy stent 210 to securely wrap and hold the thrombus therein, thereby greatly reducing the risk of distal escape the thrombus during transport within the blood vessel.

As shown in Fig. 8d, after the target thrombus 20 is transported by the thrombectomy stent 210 to a location near a distal opening 110a of the sheath 110, the external aspiration device may be used to create a continuous negative pressure in the interior of the sheath 110, under the action of which, the target thrombus 20 is sucked into the interior of the sheath 110. Finally, the thrombectomy stent 210 and the thrombus are together received into the sheath 110 and then withdrawn along with the sheath 110 out of the body. After the surgical procedure is completed, all the components are together withdrawn from the patient's body.

### <Embodiment 2>

In a second embodiment of the present invention, there is provided a thrombectomy device 200, which is substantially similar to that of the first embodiment in terms of structure, except for some differences. In the following, only these differences will be described, and the similarities between the two embodiments are not repeated.

Differing from the first embodiment, in the thrombectomy device 200 of the second embodiment, an outer mesh stent 212 is a braided stent and an inner mesh stent 211 is a cut stent. When expanded, the inner mesh stent 211 is slightly smaller in contour than, or comes into contact with, the outer mesh stent 212. This design can reduce possible damage to a blood vessel.

### <Embodiment 3>

Referring to Fig. 9, in a third embodiment of the present invention, there is provided a thrombectomy device 200, which is substantially similar to that of the first embodiment in terms of structure, except for some differences. In the following, only these differences will be described, and the similarities between the two embodiments are not repeated.

As shown in Fig. 9, in the thrombectomy device 200 of the third embodiment, an outer mesh stent 212 is provided with a graft 230 which covers some mesh openings in the outer mesh stent 212 while not blocking blood flow therethrough. More specifically, the graft 230 is disposed on a distal tapered portion 2123 of the outer mesh stent 212 to cover some mesh openings in the distal tapered portion 2123, with some mesh openings in the distal tapered portion 2123 being uncovered by the graft 230. Therefore, blood can flow through. With this arrangement, disrupted thrombus can be additionally collected and blocked from escape, imparting improved thrombectomy performance. At the same time, smooth blood flow can be attained in a lumen.

### <Embodiment 4>

Referring to Fig. 10, in a fourth embodiment of the present invention, there is provided a thrombectomy device 200, which is substantially similar to that of the first embodiment in terms of structure, except for some differences. In the following, only these differences will be described, and the similarities between the two embodiments are not repeated.

As shown in Fig. 10, in the thrombectomy device 200 of the fourth embodiment, there are multiple thrombectomy stents 210, which are integrally joined along an axis of the thrombectomy device 200 to form multiple filter mesh segments. Each thrombectomy stent 210 provides a respective one of the filter mesh segments. In this embodiment, each thrombectomy stent 210 is substantially the same as that of any preceding embodiment and, therefore, need not be described in further detail herein.

In adjacent thrombectomy stents 210, a distal end of a leading one of the thrombectomy stents 210 is coupled to a proximal end of a trailing one of them. The most distal one of the thrombectomy stents 210 is distally attached to a guide head 223, or to a movement control member 221, and the most proximal one of the thrombectomy stents 210 is proximally attached in the same manner as described in any foregoing embodiment. In this way, the movement control member 221 can be manipulated to cause simultaneous diameter changes in all the thrombectomy stents 210.

Fig. 10 only schematically illustrates the configuration when multiple outer mesh stents 212 are arranged sequentially. As can be seen, the outer mesh stents 212 are joined into a single integral piece along the axis of the thrombectomy device 200. Only the most proximal one of the outer mesh stents 212 is proximally attached to a mounting member 222, while other outer mesh stents 212 are not connected to the mounting member 222. That is, other outer mesh stents 212 are attached to the mounting member 222 via the most proximal one. The most distal outer mesh stent 212 is distally attached to the movement control member 221.

In the illustrated implementation, the most proximal outer mesh stent 212 is proximally secured to the mounting member 222 by a radiopaque ring 226, and the most distal outer mesh stent 212 is distally secured to the guide head 223. In adjacent thrombectomy stents 210, a distal connecting portion 2125 of a leading one of the outer mesh stents 212 is joined to extension struts 2121 of a trailing one of them and thereby forms an outer connecting portion 2129. Although only the attachment of the outer mesh stents 212 has been described, it will be recognized that in adjacent inner mesh stents 211, a distal extension part 2115 of a leading one of the inner mesh stents 211 may be joined to pulling struts 2111 of a trailing one of them and thereby forms an inner connecting portion. The outer connecting portions 2129 and the inner connecting portions are thinner and sparse, imparting increased compliance and adaptation of the thrombectomy device 200 in a tortuous blood vessel, while making the thrombectomy device 200 overall more resistant to bending and inward collapse in a curved blood vessel. As a result, the thrombectomy device 210 is more powerful in cutting and capturing a thrombus. The thrombectomy stents 210 can remove a thrombus in a segment-wise manner from the body. The number of thrombectomy stents 210 depends on a length of the thrombus and a thrombectomy length of each thrombectomy stent 210. With this arrangement, rapid removal of a large amount of thrombus can be achieved.

Optionally, mesh opening density of the thrombectomy stents 210 may gradually increase in the proximal-to-distal direction. Dense mesh openings can effectively block the passage of small thrombus therethrough.

Finally, it should also be noted that the thrombectomy apparatus and thrombectomy device(s) 200 in any of the above embodiments can also be used for stone removal from a bile duct or ureter, through moving the thrombectomy device 200 within the duct, stone(s) inside the duct can be extracted and collected within the mesh basket. Dense mesh openings around the distal end of the inner mesh stent 212 can effectively block small stone therethrough. In particular, when the thrombectomy device 200 includes a plurality of filter mesh segments which are arranged coaxially to form multi-layered stone removal structure, a target lumen can be repeatedly cleaned to prevent missed stones, resulting in improved stone removal efficiency.

To sum up, the thrombectomy apparatus and device of the present invention has at least the following advantages:
First, the double-layer design of the thrombectomy stent enables it to remove both old occlusive material that is hard and fresh occlusive material that is soft, resulting in improved thrombectomy efficiency and success. Meanwhile, a diameter of the thrombectomy stent in its expanded configuration can be adjusted in real time according to a varying diameter of a vascular lumen, enabling the thrombectomy stent to accommodate various conditions of the lumen. This can reduce possible damage to the blood vessel, while ensuring close contact of the outer mesh stent with a wall of the blood vessel.
Second, in case of the outer mesh stent being implemented as a cut stent, it can provide strong radial support, enabling better removal of hard occlusive material that adheres to a luminal wall. At the same time, the inner mesh stent ensures good compliance and adaptation and provides dense mesh openings within the thrombectomy stent. With this arrangement, during retraction of the thrombectomy device, a large thrombus can be cut in and disrupted by the outer mesh stent, which are then passed into the inner mesh stent, the denser mesh openings of which can prevent escape of the thrombus captured by the outer mesh stent, avoiding occlusion that may be otherwise caused.
Third, when the inner and outer mesh stents are structured so as to be larger in the middle and smaller at both ends when expanded, the thrombectomy stent can be bettered guided into the delivery device, reducing effort required to retract the thrombectomy stent. This enables more stable and easier operations during a surgical procedure.

Further, a plurality of radiopaque markers may be provided on the outer mesh body of the outer mesh stent, that are evenly spaced on a single circumference when the outer mesh stent is fully expanded. These radiopaque markers allow monitoring of how the thrombectomy stent contacts with occlusive material during a surgical procedure, facilitating adjustment of the location of the stent and its diameter in an expanded configuration for better capture and removal of the occlusive material.

Finally, the design with multiple filter mesh segments can increase the compliance and adaptation of the thrombectomy device in a tortuous lumen, eventually improving its ability to cut and capture a thrombus. Moreover, the thrombectomy stents can remove occlusive material in a segment-wise manner from the body. In this way, rapid removal of a large amount of occlusive material can be achieved with higher efficiency.

Further, it will be recognized that while the invention has been described above with reference to preferred embodiments thereof, it is not intended to be limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A thrombectomy device, comprising a thrombectomy component and a control component, wherein the thrombectomy component comprises a thrombectomy stent, wherein the thrombectomy stent comprises an outer mesh stent and an inner mesh stent nested within the outer mesh stent, wherein the control component comprises a movement control member and a mounting member, wherein the movement control member is passed through the mounting member and extends out of a distal end of the mounting member and is then coupled to a distal end of the thrombectomy stent, wherein a proximal end of the outer mesh stent is attached to the mounting member, wherein a proximal end of the inner mesh stent is attached to the mounting member or to the movement control member, wherein the movement control member is able to control the thrombectomy stent to change a diameter thereof during movement of the movement control member toward a proximal end or a distal end of the thrombectomy device.

2. The thrombectomy device according to claim 1, wherein the outer mesh stent is a cut stent, and wherein the inner mesh stent is a braided stent.

3. The thrombectomy device according to claim 1 or 2, wherein the movement control member comprises a rod-like structure and an internal luminal channel axially extending therethrough.

4. The thrombectomy device according to claim 3, wherein a proximal end of the mounting member is provided with a handle, and wherein a proximal end of the movement control member is slidably coupled to the handle.

5. The thrombectomy device according to claim 4, wherein the handle is provided with a slidable control portion, wherein the proximal end of the movement control member is coupled to the slidable control portion, and wherein the slidable control portion is configured to drive the movement control member to move.

6. The thrombectomy device according to claim 5, wherein the slidable control portion comprises a locking structure configured to restrict a movement of the movement control member, wherein the movement control member can be restricted at a plurality of positions by the locking structure in a direction of movement, and wherein the plurality of positions corresponds to different diameters of the thrombectomy stent.

7. The thrombectomy device according to claim 6, wherein the slidable control portion further comprises a slider and a slide track, wherein the slider is confined on the slide track so as to move along the slide track, wherein the proximal end of the movement control member is coupled to the slider, wherein the locking structure comprises a first locking member arranged on the slider and a plurality of second locking members arranged on the slide track, wherein the plurality of second locking members are arranged along a movement direction of the slider, and wherein the first locking member is selectively engageable with at last one of the plurality of the second locking members to restrict a movement of the slider.

8. The thrombectomy device according to claim 1 or 2, wherein the thrombectomy stent, when expanded, has a diameter increasing and then decreasing from a proximal end to a distal end and has a maximum diameter greater than or equal to a diameter of a target lumen.

9. The thrombectomy device according to claim 8, wherein the outer mesh stent comprises a proximal connecting portion, a proximal tapered portion, an intermediate wall-contacting portion, a distal tapered portion and a distal connecting portion that are joined in sequence from a proximal end to a distal end along an axis of the outer mesh stent, wherein the proximal connecting portion is made up of a plurality of extension struts that are circumferentially arranged, wherein the plurality of extension struts are coupled to the mounting member, wherein the proximal tapered portion has a diameter gradually increasing from a proximal end to a distal end along the axis of the outer mesh stent, wherein the distal tapered portion has a diameter gradually decreasing from a proximal end to a distal end along the axis of the outer mesh stent, wherein the intermediate wall-contacting portion has a maximum diameter greater than or equal to the diameter of the target lumen, wherein a diameter of the distal connecting portion is smaller than the diameter of the distal tapered portion, and wherein the distal tapered portion is coupled to the movement control member.

10. The thrombectomy device according to claim 8, wherein the inner mesh stent comprises a proximal extension part, a proximal mesh surface, a distal mesh surface and a distal extension part that are joined in sequence from a proximal end to a distal end along an axis of the inner mesh stent, wherein the proximal extension part is made up of a plurality of pulling struts that are circumferentially arranged, wherein the plurality of pulling struts are coupled to the mounting member or to the movement control member, wherein the proximal mesh surface has a diameter gradually increasing from a proximal end to a distal end along the axis of the inner mesh stent, wherein the distal mesh surface has a diameter gradually decreasing from a proximal end to a distal end along the axis of the inner mesh stent, wherein a diameter of the distal extension part is diameter smaller than the diameter of the distal mesh surface, and wherein the distal extension part is coupled to the movement control member.

11. The thrombectomy device according to claim 10, wherein a mesh opening density of the proximal mesh surface is lower than a mesh opening density of the distal mesh surface, wherein a proximal end of the proximal extension part is inwardly crimped onto the outer surface of the movement control member, and wherein the distal extension part is inwardly crimped onto the outer surface of the movement control member.

12. The thrombectomy device according to claim 1 or 2, wherein radiopaque markers are provided at a maximum diameter of an expanded outer mesh stent, and wherein the plurality of radiopaque markers are provided on a single circumference of the outer mesh stent.

13. The thrombectomy device according to claim 1 or 2, wherein the thrombectomy component comprises a single or a plurality of thrombectomy stents, wherein in case of a plurality of thrombectomy stents, the plurality of thrombectomy stents are arranged in sequence along an axis of the thrombectomy device and are connected as a single piece, wherein after being passed out of the distal end of the mounting member, the movement control member is successively through all the thrombectomy stents and is coupled to a distal end of a distal-most one of the thrombectomy stents, and wherein a proximal end of a proximal-most one of the thrombectomy stents is coupled to the control component.

14. The thrombectomy device according to claim 13, wherein a mesh opening density of the plurality of thrombectomy stents gradually increases from a proximal end to a distal end.

15. The thrombectomy device according to claim 13, wherein in case of a single thrombectomy stent, the thrombectomy stent is coupled at a proximal end and a distal end to the control component through radiopaque rings, and wherein in case of a plurality of thrombectomy stents, a proximal end of a proximal-most one of the thrombectomy stents is coupled to the control component through a radiopaque ring, and wherein a distal end of a distal-most one of the thrombectomy stents is coupled to the control component through a radiopaque ring.

16. The thrombectomy device according to claim 1 or 2, wherein the thrombectomy component further comprises a graft covering some mesh openings of the outer mesh stent.

17. A thrombectomy apparatus, comprising a delivery device, an aspiration catheter and the thrombectomy device of any one of claims 1 to 16, wherein the delivery device is configured to deliver the thrombectomy device to a target thrombectomy site, wherein the aspiration catheter is coupled to the delivery device and is configured to apply a suction force via the delivery device for aspirating occlusive material in a target lumen.
